# EUROPEAN PATENT APPLICATION

(11) **EP 1 728 858 A1**
(43) Date of publication of application: **06.12.2006**
(21) Application number: 05291167.4
(22) Date of filing: 31.05.2005
(51) Int. Cl.: C12N 5/08, C12N 5/06, G01N 33/50

(54) **Primitive endodermal stem cells, a process for preparing them and their use, in particular for obtaining primitive epithelial liver cells**

(71) Applicant: Abcys S. A., 75010 Paris (FR)
(72) Inventor: Hatzfeld, Jacques, 92160 Antony (FR); Laabi, Yacine, 94230 Cachan (FR); Herpe, Yves-Edouard, 75013 Paris (FR); Barbet, Romain, 94800 Villejuif (FR)
(74) Representative: Grosset-Fournier, Chantal Catherine

(57) **Abstract**

The invention relates to the use of a cell line for deriving human primitive endodermal stem cells and / or human primitive epithelial liver cells and / or human matured epithelial liver cells in vitro from human pluripotent stem cells, in particular from human embryonic stem cells.

## Description

The invention relates to primitive endodermal stem cells, a process for preparing them and their use, in particular for obtaining primitive epithelial liver cells.

The invention more particularly relates to primitive endodermal stem cells and primitive epithelial liver cells derived from human embryonic stem cells.

Obtaining normal human cells is currently a critical problem because the number of organ donors for research purposes is very low. An alternative for obtaining normal human cells is the use of human embryonic stem cells. These human embryonic stem cells are indeed pluripotent, which means that they are able to differentiate *in vitro* into all the different tissues of the human body. Thus they are a unique research tool which enables to obtain any kind of human cell, which could ultimately contribute to solve the problem posed by the rarity of organs from human donors.

However, obtaining differentiated tissues *in vitro* from human embryonic stem cells remains a major scientific challenge, because the molecular mechanisms which control the biological processes involved are poorly known.

Among the possible differentiation pathways for human embryonic stem cells, the hepatic pathway is of major interest for various applications such as drug-screening, toxicological studies or cell therapy. During embryogenesis, formation of the liver occurs via successive steps of cell differentiation :
1) differentiation into endodermal cells (the endoderm being at the origin of pancreas, lung, intestine, thymus, liver, etc.),
2) differentiation into primitive hepatic stem cells (hepatoblasts or oval cells)
3) differentiation into mature liver cells, i.e. hepatocytes on the one hand and cholangiocytes on the other hand.

Regarding the *in vitro* differentiation of human embryonic stem cells along the hepatic lineage, the few results obtained so far are not satisfactory, because differentiation seems to occur spontaneously and randomly.

Rambhatla et al. (Cell Transplantation 12:1-11, 2003) disclose a method for deriving hepatic cells from human embryonic stem cells, either directly or *via* the formation of embryoid bodies. They use specific differentiation factors such as sodium butyrate and DMSO (dimethyl-sulfoxide) or trichostatine A. It has to be noted that the yield of hepatic differentiation remains relatively low (10-15 %). International application WO 01/81549 relates to this differentiation method.

Another differentiation method was disclosed by Shirahashi et al. (Cell Transplantation 13:197-211, 2004). In their method, human or murine embryonic stem cells are differentiated along the hepatic lineage by culturing stem cells on a fibroblast layer, obtaining embryoid bodies, and then placing these embryoid bodies in particular culture conditions, notably in the presence of human insulin and dexamethasone. Differentiation seems to occur between day 17 and 43 after the initial seeding of the stem cells. The yield in differentiated cells is not specified.

Lavon et al. (Differentiation, 72:230-238, 2004) describe yet another method of differentiation along the hepatic lineage. This method consists in transfecting human embryonic stem cells with a hybrid albumin-eGFP (enhanced green fluorescent protein) gene, culturing the cells so as to obtain embryoid bodies and isolating those of the cells which express GFP by FACS (fluorescence activated cell sorter) analysis. The yield of the differentiation process is only 6 %, and differentiation into liver cells occurs no sooner than day 30. Besides, the liver cells obtained cannot be considered as normal liver cells since their genome has been modified in the process.

Some of the principal disadvantages of currently available methods of differentiation along the hepatic lineage are their poor yield in terms of differentiated cells (which also means that the populations of cells obtained by those methods are very heterogeneous), the necessity of deriving hepatic cells *via* embryoid bodies in most of the cases and the spontaneity and randomness of the differentiation.

Deriving hepatic cells *via* embryoid bodies is disadvantageous in that an embryoid body is a mixture of different cell types in which the population of interest is very diluted ; an additional purification step is thus necessary to finally obtain the differentiated hepatic cells.

By "spontaneity and randomness" is meant that the differentiation is poorly controlled, poorly reproducible and / or rather unpredictable.

Besides there has been no available method so far for deriving cells at different definite stages of the development of the liver, such as primitive endodermal cells, liver stem cells, hepatoblasts, hepatocytes, etc. For instance, the fact that alpha-fetoprotein is not expressed by the cells obtained according to any one of the three above-mentioned methods means that only hepatocytes, not hepatoblasts or oval cells, are obtained.

### Description of the invention

One object of the invention is to provide a population of human primitive endodermal stem cells massively and rapidly derived *in vitro* from human embryonic stem cells.

Another object of the invention is to provide a homogenous population of primitive epithelial liver stem cells and a population of matured epithelial liver cells differentiated therefrom.

It is another object of the invention to provide a method for deriving the above-mentioned populations of cells from human embryonic stem cells in a controlled, well characterized, reproducible way, without any need for transfection nor for prior formation of embryoid bodies.

Another object yet of the invention is the use of the above-mentioned populations of cells or of cells from said populations for carrying out drug-screening or toxicological tests or for preparing pharmaceutical compositions.

The different objects of the invention are achieved by using the YL-1 (SCL-19) cell-line, deposited at CNCM under No. 1-3348 on December 23, 2004, said cell-line being unexpectedly able to express one or several factors which can induce the massive differentiation of human embryonic stem cells into primitive endodermal stem cells, which then serve as a basis for further differentiation into primitive epithelial liver stem cells and matured epithelial liver cells.

The invention relates to the use of the YL-1 (SCL-19) cell line, deposited at CNCM under No. I-3348 on December 23, 2004, for deriving human primitive endodermal stem cells and / or human primitive epithelial liver cells and / or human matured epithelial liver cells in vitro from human pluripotent stem cells, in particular from human embryonic stem cells.

By "pluripotent stem cells" is meant cells derived from pre-embryonic, embryonic or fetal tissue at any time after fertilization and having the characteristic of being capable under the right conditions to self-renew and produce progeny of different cell types. These pluripotent cells can give rise to progeny that are derivatives of the three embryonic germ layers, mesoderm, ectoderm and endoderm. Non-limiting examples of these pluripotent stem cells are human embryonic stem cells, stem cells derived from placenta, umbilical cord blood and bone marrow.

By "human embryonic stem cells" is meant cells derived from the inner cell mass of a human blastocyst, which can self-renew indefinitely under defined conditions of cell culture and which are pluripotential in the sense that they can give rise, under specific conditions of cell culture, to the three embryonic germ layers, namely endoderm, mesoderm and ectoderm. These three germ layers will participate in the formation of all the cells which compose a human fetus and / or an adult human body.

By "human primitive endodermal stem cells" is meant stem cells which are at the origin of the endodermal component of embryonic, fetal and adult organs such as liver, pancreas, gut, thymus, lung etc.

By "human primitive epithelial liver cells" is meant stem cells that are in general bi-potential, such as hepatoblasts or oval cells, which means that they can give rise under suitable environmental conditions to both hepatocytic and bile duct (cholangiocytic) cell lineages.

By "human matured epithelial liver cells" is meant differentiated cells having morphological and phenotypic characteristics of hepatocytes or cholangiocytes.

A complete description of the morphology and the phenotype of the cells is available in the examples.

The invention also relates to a population of human primitive endodermal stem cells, containing cells expressing albumin, alpha-fetoprotein, and at least one of GATA-6 and SOX-17.

By "population of cells" is meant a set of cells exhibiting at least partially homogenous features. The above-mentioned population of human primitive endodermal cells may contain various cells, provided that at least some of these cells express albumin, alpha-fetoprotein and GATA-6 at the same time or at least some of these cells express albumin, alpha-fetoprotein and SOX-17 at the same time. The above-mentioned population may also contain cells that express albumin, alpha-fetoprotein, GATA-6 and SOX-17 at the same time.

SOX-17 and GATA-6 are endoderm-specific transcription factors whose gene knock-out in the mouse impairs definitive endoderm formation, whereas alpha-fetoprotein and albumin are intracellular and secreted proteins whose expression is usually correlated with a liver cell phenotype.

The invention more particularly relates to a population of human primitive endodermal stem cells, containing cells expressing albumin, alpha-fetoprotein, and at least one of GATA-6 and SOX-17, wherein the SSEA-3 marker is down-regulated relative to undifferentiated pluripotent human embryonic stem cells.

SSEA-3 is a typical cell surface marker known to be expressed only by pluripotent self-renewing embryonic stem cells.

By "down-regulated" is meant that a progressive decrease of protein expression as measured by flow cytometry until reaching a complete extinction.

According to a particular embodiment, at least 90 % of the cells of said population express albumin and at least 90 % of the cells of said population express alpha-fetoprotein and at least about 40 % of the cells of said population express GATA-6 and at least about 40 % of the cells of said population express SOX-17.

The above-mentioned population of human primitive endodermal stem cells, containing cells expressing albumin, alpha-fetoprotein, and at least one of GATA-6 and SOX-17, advantageously comprises cells which express one or more of the following markers: CD29, CD49f, CD54 and CD56.

CD29 is also called β1-integrin and is the receptor of fibronectine. Associated with CD49f it constitutes the receptor of laminin.

CD49f is also called α6-integrin.

CD29 et CD49f are used as surface markers for phenotypically characterize mouse and rat fetal hepatoblats (Suzuki, A. et al, J.C.B., 2002, 156, 1: 173-184)

CD54 is also called I-CAMI (intercellular cell adhesion molecule-1) and participates in cell adhesion.

CD56 is also called N-CAM (neural cell adhesion molecule 1) and is a membrane glycoprotein which plays a role in cell-cell adhesion or in cell-extracellular matrix adhesion. It also takes part in morphogenesis during the embryonic development. This protein is used here because it is not expressed by human embryonic stem cells which are under conditions of self-renewal.

More advantageously, said population of human primitive endodermal stem cells comprises cells which express CD29, CD49f, CD54 and CD56.

In another particular embodiment of the invention the above-mentioned population of human primitive endodermal stem cells is further characterized in that :
more than about 50 %, in particular from about 70 % to about 80 %, in particular about 75 % of the cells of said population express CD29 and / or
more than about 60 %, in particular from about 70 % to about 85 %, in particular about 75 % of the cells of said population express CD49f and / or
more than about 1 %, in particular from about 1 % to about 3 %, in particular about 2 % of the cells of said population express CD54 and /or
more than about 15 %, in particular from about 40 % to about 60 %, in particular about 50 % of the cells of said population express CD56.

The invention also relates to a population of human primitive epithelial liver stem cells, which is substantially homogeneous and wherein the cells of said population have the property of being able to multiply without any substantial change of phenotype for at least 20 passages and at least part of those cells express alpha-fetoprotein, albumin, CK18, CD56 and c-met.

By "substantially homogeneous" is meant a population of cells in which the cells display a similar morphology and are all refringent under a phase contrast microscope. A typical morphology of this population of cells is described in more detail in examples 5 and 6.

By "able to multiply for at least 20 passages" is meant able to multiply for at least about 20 weeks, since cells are generally passaged once a week. 20 passages usually correspond to more than 20 cell divisions.

By "without any substantial change of phenotype" is meant without a significant change in the amount or intensity of surface markers or intracellular proteins expressed in the cells.

The following markers are also advantageously expressed by this population of human primitive epithelial liver stem cells : CK8, alpha-AAT, vimentin, SOX-17, CD49f, CD29, CD54 and CD90.

CK8 and CK18 are cytokeratins, i.e. proteins from the family of intermediate filaments of the cytoskeleton that are expressed in epitheliums of various origin.

Alpha-AAT is a serum protein which is synthesized and secreted by the liver.

Vimentin is a protein from the family of intermediate filaments.

c-met is the receptor of the hepatocyte growth factor (HGF) which plays a major role in the process of liver development from the endoderm but also at later stages, such as the proliferation of hepatic stem cells.

CD90 or Thy-1 is a marker which is often found in populations of stem cells such as hematopoietic stem cells.

The invention also relates to a population of human matured epithelial liver cells, wherein albumin is substantially expressed by cells of said population and alpha-fetoprotein is substantially not expressed by cells of said population.

By "substantially expressed" is meant yielding a positive result for immunocytochemistry and / or immunohistochemistry.

By "substantially not expressed" is meant yielding a negative result for immunocytochemistry and / or immunohistochemistry.

The invention also relates to a human primitive endodermal stem cell, characterized in that it expresses alpha-fetoprotein, albumin, SOX-17 and GATA-6.

In a preferred embodiment, said human primitive endodermal stem cell which expresses alpha-fetoprotein, albumin, SOX-17 and GATA-6 is further characterized in that it expresses one or more of the following markers : CD29, CD49f, CD54 and CD56.

Said human primitive endodermal stem cell is advantageously further characterized in that it expresses alpha-fetoprotein, albumin, SOX-17, GATA-6, CD29, CD49f, CD54 and CD56.

The invention also relates to a human primitive epithelial liver stem cell, characterized in that it expresses alpha-fetoprotein, albumin, CK18, CD56 and c-met.

Another aspect of the invention is a process for preparing primitive endodermal stem cells in vitro, comprising :
- the incubation of a culture of human embryonic stem cells in a YL-1 (SCL-19) conditioned medium derived from a culture of YL-1 (SCL-19) cells from the YL-1 (SCL-19) cell line deposited at CNCM under No. 1-3348 on December 23, 2004, for an amount of time appropriate for the differentiation of said human embryonic stem cells into primitive endodermal stem cells, and
- the recovery of said primitive endodermal stem cells from said YL-1 (SCL-19) conditioned medium.

Said recovery can be done by dissecting the three-dimensional structures described in example 1 under a dissection microscope and mechanically dissociating the cells into small clumps.

A possible basis for determining whether primitive endodermal stem cells have been obtained is a morphological observation, such as the observation of tridimensional crest structures (see example 1).

In a particular embodiment, the YL-1 (SCL-19) conditioned medium of said process is obtained by :
- irradiating a culture of YL-1 (SCL-19) cells from the YL-1 (SCL-19) cell line deposited at CNCM under No. I-3348 on December 23, 2004,
- further culturing the YL-1 (SCL-19) cells in a primary YL-1 (SCL-19) culture medium to obtain a YL-1 (SCL-19) conditioned medium, and
- recovering said YL-1 (SCL-19) conditioned medium.

By "irradiating" is meant treating by exposure to radiation such as gamma radiation. This treatment inhibits cell proliferation but does not affect the synthesis and secretion of factors important for human embryonic stem cell differentiation into endoderm.

The amount of time appropriate for the differentiation of said human embryonic stem cells into primitive endodermal stem cells is particularly at least 4 days, in particular 4 to 20 days.

In a preferred embodiment the above-mentioned process comprises :
- the culture of human embryonic stem cells in KOSR medium,
- the incubation of a sample of said human embryonic stem cells on a culture of mitomycin-treated mouse embryonic fibroblasts in the YL-1 (SCL-19) conditioned medium, said YL-1 (SCL-19) conditioned medium being obtained by
   - seeding YL-1 (SCL-19) cells from the YL-1 (SCL-19) cell line deposited at CNCM under No. I-3348 on December 23, 2004 in a primary YL-1 (SCL-19) culture medium such as the one comprising 90 % Dulbecco's modified Eagle's medium, 10 % fetal calf serum, 1 mM glutamine and 0.1 mM betamercaptoethanol,
   - irradiating by gamma irradiation said YL-1 (SCL-19) cells,
   - replacing said YL-1 (SCL-19) culture medium by KOSR medium,
   - filtering said KOSR medium after about 2 days after said seeding,
   said incubation lasting for an amount of time appropriate for the differentiation of said human embryonic stem cells into primitive endodermal stem cells, said amount of time being at least 4 days, in particular 4 to 20 days, and
- the recovery of said primitive endodermal stem cells from said YL-1 (SCL-19) conditioned medium.

The KOSR medium is described in the experimental section below.

It has to be noted that the differentiation into endodermal cells may be under way before the 4^{th} day of incubation, even though no morphological sign of this differentiation can yet be observed.

If the stem cells are incubated for more than 20 days, the cell culture is usually too confluent and there are usually too many dead cells for the process to be carried on.

The invention also provides a YL-1 (SCL-19) conditioned medium such as obtained by :
- irradiating a culture of YL-1 (SCL-19) cells from the YL-1 (SCL-19) cell line deposited at CNCM under No. I-3348 on December 23, 2004,
- further culturing the YL-1 (SCL-19) cells in a primary YL-1 (SCL-19) culture medium to obtain a YL-1 (SCL-19) conditioned medium, and
- recovering said YL-1 (SCL-19) conditioned medium.

This YL-1 (SCL-19) conditioned medium can in particular be used for preparing human primitive endodermal stem cells in vitro from human embryonic stem cells using the above-mentioned process.

The invention also relates to a population of primitive endodermal stem cells such as prepared in vitro according to the above-mentioned process.

The invention also provides a process for preparing primitive epithelial liver stem cells in vitro, comprising :
- the culture of primitive endodermal stem cells prepared according to the above-mentioned process for preparing primitive endodermal stem cells in vitro for a sufficient amount of time in the presence of the MCM medium so that said primitive endodermal stem cells differentiate into primitive epithelial liver stem cells, and
- the recovery of said primitive epithelial liver stem cells from said MCM medium.

A possible basis for determining whether primitive epithelial liver stem cells have been obtained is a morphological observation and phenotypic observation, as described in examples 5 and 6.

In a particular embodiment said MCM medium is obtained through a process comprising culturing fibroblasts in a culture medium and then recovering said culture medium.

Said MCM medium is advantageously such as obtained by the following process comprising:
- propagating mouse embyronic fibroblasts in a medium containing 90 % Dulbecco's modified Eagle's medium, 10 % fetal calf serum, 1 mM sodium pyruvate, and 2 mM glutamine,
- exposing said fibroblasts to 10 µg/ml ametycin,
- washing said fibroblasts,
- exposing said fibroblasts to a trypsin / EDTA mixture,
- resuspending said fibroblasts in a medium containing 90 % Dulbecco's modified Eagle's medium, 10 % fetal calf serum, 1 mM sodium pyruvate, and 2 mM glutamine,
- plating said fibroblasts,
- after 24 hours, changing the medium to KOSR medium,
- after 1 more day, changing the medium to KOSR medium supplemented with bFGF (basic fibroblast growth factor), and
- after 1 more day, collecting and filtering the medium.

In another particular embodiment the process for preparing primitive epithelial liver stem cells in vitro is such that said sufficient amount of time is approximately one week.

The process for preparing primitive epithelial liver stem cells in vitro advantageously comprises :
- the preparation of primitive endodermal stem cells according to the above-mentioned process for preparing primitive endodermal stem cells in vitro,
- the treatment of said primitive endodermal stem cells with dissociating means, the seeding of said cells on culture plates coated with collagen in the MCM medium,
- the culture of said primitive endodermal stem cells for at least one week, so that said primitive endodermal stem cells differentiate into primitive epithelial liver stem cells,
- the optional passaging of said primitive epithelial liver stem cells once or several times using a detaching means such as DispaseII and said MCM culture medium, and
- the recovery of said primitive epithelial liver stem cells from said MCM medium.

By "dissociating means" is in particular meant means for mechanical dispersion into small clumps as described above. Alternatively, said dissociating means could also be of chemical or biochemical origin.

The "detaching means" has the same function as the dissociation means but generally consists in the use of a chemical or biochemical substance such as DispaseII.

The invention also relates to a population of primitive epithelial liver stem cells such as prepared according to the above-mentioned process for preparing primitive epithelial liver stem cells in vitro.

The invention also relates to a process for preparing matured epithelial liver cells in vitro, comprising :
- the culture of primitive epithelial liver stem cells prepared according to the above-mentioned process for preparing primitive epithelial liver stem cells in vitro in a maturation medium so that said primitive epithelial liver stem cells differentiate into matured epithelial liver cells, and
- the recovery of said matured epithelial liver cells from said maturation medium.

A possible basis for determining whether matured liver stem cells have been obtained is a morphological observation, as described in examples 7. Matured liver stem cells are also characterized in that they are less refringent when observed using a phase contrast microscope relative to primitive epithelial liver stem cells.

In a particular embodiment said process for preparing matured epithelial liver cells in vitro comprises :
- the preparation of primitive epithelial liver stem cells according to the above-mentioned process for preparing primitive epithelial liver stem cells in vitro,
- the culture of said primitive epithelial liver stem cells in a maturation medium comprising 90 % Dulbecco's modified Eagle's medium, 10 % fetal calf serum, 1 % non-essential amino-acids, 1 mM sodium pyruvate, 2 mM glutamine, 1% insulin / transferrin / selenium, so that said primitive epithelial liver stem cells differentiate into matured epithelial liver cells, and
- the recovery of said matured epithelial liver cells from said maturation medium.

The invention also relates to population of matured epithelial liver cells such as prepared according to the above-mentioned process for preparing matured epithelial liver cells in vitro.

The invention also relates to the use of a population of cells, said population of cells being the above-mentioned population of primitive endodermal stem cells or the above-mentioned population of primitive epithelial liver stem cells or the above-mentioned population of matured liver stem cells, or of the above-mentioned cells, for carrying out toxicological tests comprising :
- submitting said population of cells or said cells to a substance having a possible toxic effect on said population of cells or said cells, and
- assessing the toxicity of said substance on said population or said cells.

By "substance having a possible toxic effect" is meant an exogenous chemical substance either of natural origin, such as a toxin, a neurotransmitter, a hormone, a molecule extracted from a plant or animal, in particular a peptide, polypeptide or nucleic acid, or obtained via a human activity, such as a drug, a medicament, a pollutant, a solvent, a xenobiotic substance. The toxic effect may consist in any perturbation of any of the physiological functions of the organism, observed at a certain dose of the substance.

Many methods can be used for assessing the toxicity of the substance on cells, among which the observation of the morphology of the cells under phase contrast or electron microscope, cytochrome P450 induction, MTT test, LDH test, transaminase leakage, plasmatic protein synthesis alteration (for example of transferrin and albumin), or any other method known by those skilled in the art for revealing a possible effect of the substance on cell viability, survival or on any function of the cells.

The assessment of the toxicity of a substance on cells of the hepatocyte lineage is particularly important for drug-screening applications, since hepatocytes are cells responsible for blood detoxification and thus elimination of harmful compounds from the organism.

The invention also relates to the use of a population of cells, said population of cells being the above-mentioned population of primitive endodermal stem cells or the above-mentioned population of primitive epithelial liver stem cells or the above-mentioned population of matured liver stem cells or of the above-mentioned cells, for carrying out drug screening comprising :
- submitting said population of cells or said cells to a substance having a possible therapeutic or prophylactic effect on humans or animals,
- either before or after said submitting to said substance, submitting said population of cells or said cells to a treatment for placing said population or said cells in a substantially pathological state, and
- assessing the possible therapeutic or prophylactic effect of said substance on said population of cells or said cells placed in a substantially pathological state.

Examples of pathologies where this use for carrying drug-screening could be useful include cancers, genetic pathologies and hepatic disorders, in particular inherited hepatic disorders such as Wilson's disease, alpha-1 antitrypsin deficiency, erythropoietic protoporphyria, Niemann-Pick disease or type I tyrosinemia and acquired hepatic disorders such as end-stage cirrhosis and liver-based congenital metabolic diseases.

This use more particularly relates to the screening of candidate pharmaceutical compounds. The assessment of the possible therapeutic or prophylactic effect of the candidate compound can be made by observing a change in the morphology, marker phenotype or metabolic activity of the cells and usually involves a comparison with untreated cells or cells treated with an inert compound.

The invention also relates to a pharmaceutical composition comprising as active substance a population of cells, said population of cells being the above-mentioned population of primitive endodermal stem cells or the above-mentioned population of primitive epithelial liver stem cells or the above-mentioned population of matured liver stem cells, or the above-mentioned cells, in association with a pharmaceutically acceptable carrier.

For human therapy the appropriate dosage is generally between 10⁹ and 10¹² cells, depending on the body weight, the nature and severity of the pathology to be treated, and the replicative capacity of the administered cells.

This pharmaceutical composition can in particular be applied for restoring liver function in a human or animal subject needing such therapy, for instance due to an acute, chronic or inherited impairment of liver function. This pharmaceutical composition may take the form of encapsulated cells or of a bioartificial liver device.

Alternatively, the cells or populations of cells of the invention, and in particular the matured liver cells, could be used for implementing a device for detoxifying a fluid such as blood by removing or altering various compounds usually processed by the liver from said fluid.

The invention also relates to the use of a population of cells, said population of cells being the above-mentioned population of primitive endodermal stem cells or the above-mentioned population of primitive epithelial liver stem cells or the above-mentioned population of matured liver stem cells, or of the above-mentioned cells, for preparing a medicament for the treatment of cancers or of genetic pathologies or of hepatic disorders, in particular for the treatment of inherited hepatic disorders such as Wilson's disease, alpha-1 antitrypsin deficiency, erythropoietic protoporphyria, Niemann-Pick disease or type I tyrosinemia or of acquired hepatic disorders such as end-stage cirrhosis and liver-based congenital metabolic diseases.

### Brief description of the figures

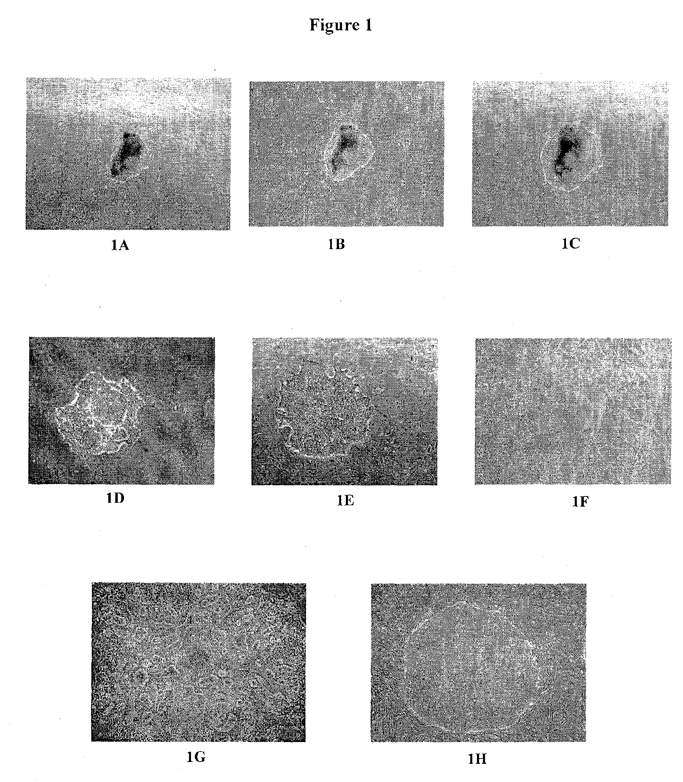

Figure 1 shows pictures taken during the differentiation process of human embryonic stem cells into human primitive endodermal stem cells.
Picture 1A : day 1 of differentiation.
Picture 1B : day 2 of differentiation.
Picture 1C : day 3 of differentiation.
Picture 1D : day 4 of differentiation.
Picture 1E : day 5 of differentiation.
Picture 1F : day 6 of differentiation.
Picture 1G : day 7 of differentiation.
Picture IH : a normal colony of human embryonic stem cells at day 7 (control).
Figure 2 shows a semi-thin section of typical colonies harboring crests (A) and cavities (B) obtained during the differentiation process from human embryonic stem cells into human primitive endodermal stem cells (see Example 1 below). Magnification : × 20.
Figure 3 shows an electron microscopy photomicrograph of human embryonic stem cells under differentiation into human primitive endodermal stem cells at day 7. A : glycogen. B : junctional complex. C : bile canaliculus ; D : microvilli.
Figures 4.1 to 4.4 show the expression of markers during differentiation of human embryonic stem cells into human primitive endodermal stem cells (see Example 3 below). The number of positive cells (Y axis) is plotted versus the number of days after the onset of the differentiation process (X axis). The continuous line with squares represents the culture in the YL-1 (SCL-19) conditioned medium, and the dashed line with circles represents the culture in a KOSR medium.

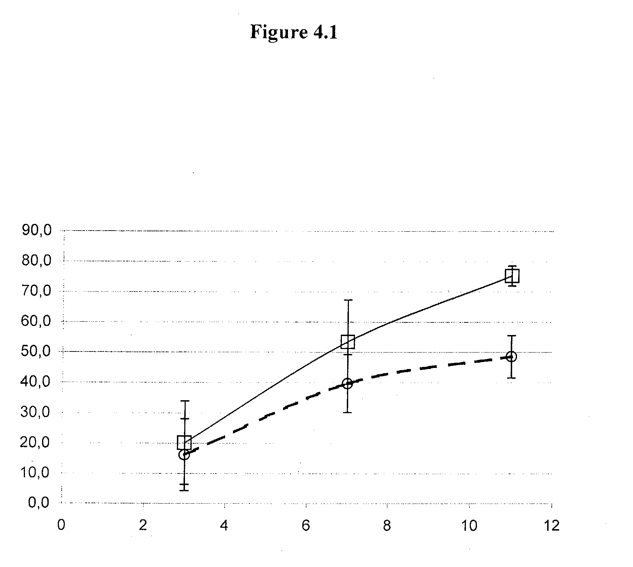

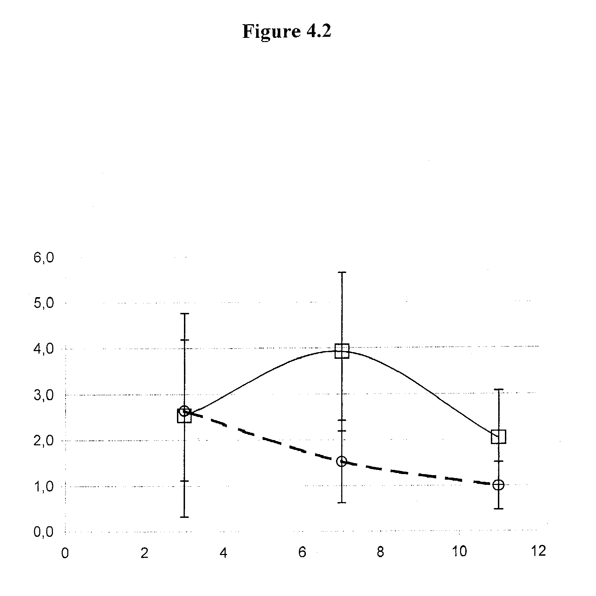

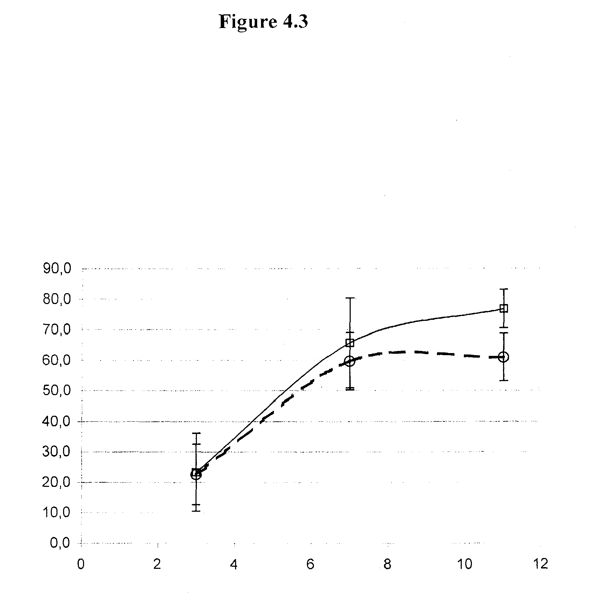

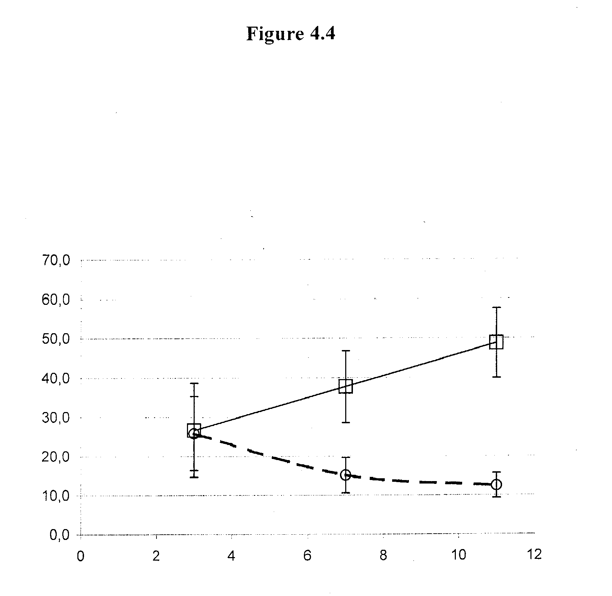

Figure 4.1 : marker CD29;
Figure 4.2 : marker CD54 ;
Figure 4.3 : marker CD49f ;
Figure 4.4 : marker CD56.

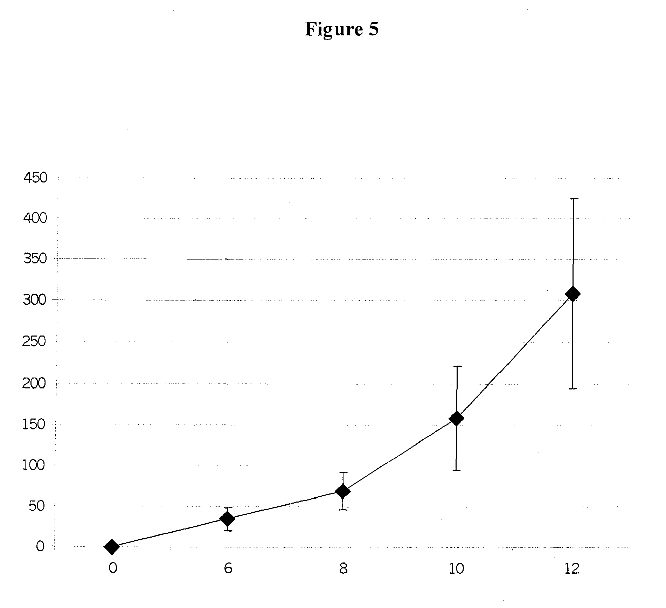

Figure 5 shows the secretion of alpha-fetoprotein by differentiating cells. The measured concentration of alpha-fetoprotein on the Y-axis in ng/ml is plotted vs. the number of days on the X-axis.

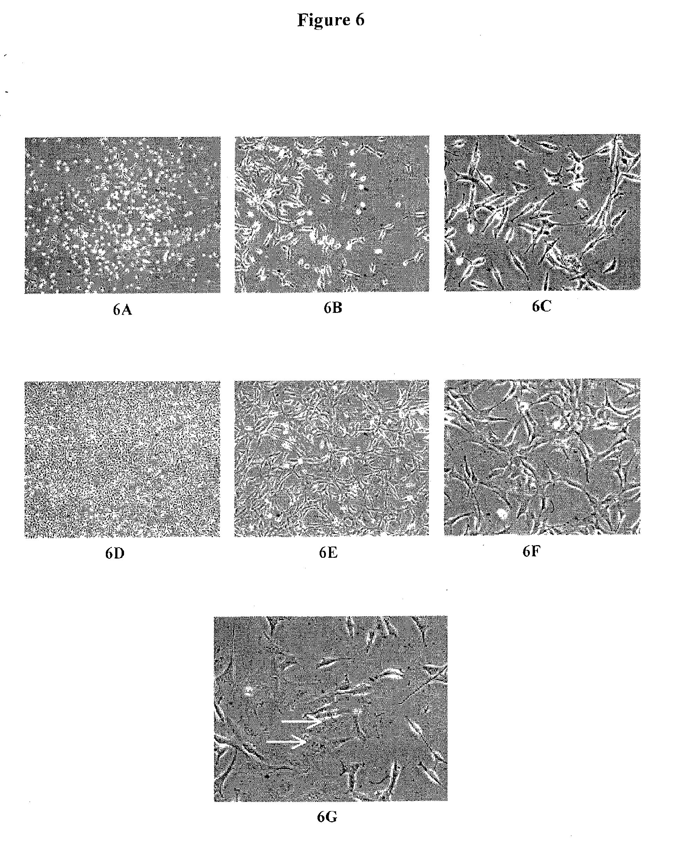

Figure 6 shows the morphology of the primitive epithelial liver stem cell line. Pictures 6A, 6B and 6C show an example of a sub-confluent culture. Pictures 6D, 6E and 6F show an example of a confluent culture. Magnification : × 4 for pictures 6A and 6D ; × 10 for pictures 6B and 6E ; × 20 for pictures 6C and 6F. Picture 6G shows an example of binucleated cells (see white arrows).

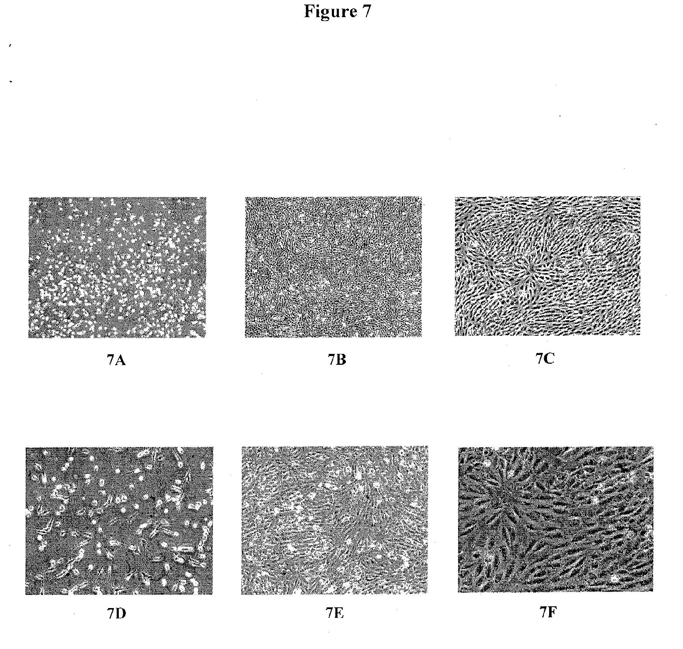

Figure 7 shows the morphology of the primitive epithelial liver stem cell line under maturation. Pictures 7A and 7D show a view of single cells. Pictures 7B and 7E show a view of a sub-confluent culture. Pictures 7C and 7F show a view of a confluent culture. Magnification : × 4 for pictures 7A, 7B and 7C ; × 10 for pictures 7D, 7E and 7F.

### Experimental section : materials and methods

This section provides details of the techniques and reagents used in the examples below.

### Media and feeder cells

Suitable sources are as follows : Dulbecco's modified Eagle's medium (DMEM) containing 4.5 g/L of glucose (Gibco), Knockout Dulbecco's modified Eagle's medium (KO DMEM) (Gibco), L-glutamine (Biological Industries), non-essential amino acids solution (Gibco), β-mercaptoethanol (Gibco), human recombinant basic fibroblast growth factor (bFGF) (Peprotech), Serum Replacement (SR) (Gibco), Fetal Calf Serum (FCS) (Sigma), sodium pyruvate (Sigma).

Serum-free human embryonic stem cells (hESC) medium (KOSR medium) is made with 80% KO DMEM, 20% SR, 1% non-essential amino acids, 1 mM glutamine and 0.1 mM β-mercaptoethanol. Just before use, human bFGF is added to a final concentration of 4 ng/ml.

Serum-containing medium for mouse embryonic fibroblasts (MEF medium) is made with 90% DMEM, 10% FCS, 1 mM sodium pyruvate and 2 mM glutamine.

All the media are filtered and stored at 4°C.

### Preparation of mitomycin-treated mouse embryonic fibroblasts

Mouse embryonic fibroblasts (MEFs) were propagated in MEF medium in T150 flasks (Corning). To prepare the feeder layer, cells were exposed to 10µg/ml of Ametycin (Sanofi-Synthélabo) for 3 hours at 37°C. This treatment inhibits cell proliferation but does not affect the synthesis of factors important for hESC self-renewal. Cells were then washed twice with PBS (phosphate buffer saline) without calcium and magnesium (Cambrex) then a single cell suspension was obtained using trypsin 0.005%/EDTA 0.01% (Biological Industries). 6-well culture plates (Falcon) were coated for 20 minutes at room temperature with 0.1% gelatin (Sigma) and plated with 4.10⁴ MEFs /cm² per well in MEF medium. These feeder cells were used within one week after plating. A few hours before hESCs seeding, the medium was replaced with fresh KOSR medium supplemented with bFGF. Cells were incubated at 37°C in humidified air incubator gassed with 5% CO₂.

### Propagation of human embryonic stem cells

hESCs were seeded as small clusters on mitomycin-treated mouse embryonic fibroblasts seeded in a 6-well culture plate in KOSR medium supplemented with bFGF. The medium was renewed every day for 7 days. For passaging, the medium was removed, colonies were washed twice with PBS then treated for 10 minutes at 37°C with collagenase IV (Gibco). Cells were then washed once with KOSR medium, small differentiated colonies were removed manually under the microscope and the colonies were scraped using a 5 ml pipette. Small clusters were discarded and the big clusters were transferred onto a mitomycin-treated feeder layer. In general, one well of a 6-well culture plate was used to seed 8 wells of a 6-well culture plate. Cells were incubated at 37°C in humidified air incubator gassed with 5% CO₂.

### Preparation of MEFs conditioned medium (MCM medium)

Mitomycin-treated MEFs were prepared as described above. After trypsinization, cells were resuspended in MEF medium and plated in a T75 flask (Falcon) at a density of 5.6.10⁴ cells /cm². 24 hours later, the medium was changed into KOSR medium. 48 hours later, the medium was changed once again with the addition of 4 ng/ml of bFGF. 72 hours later, the medium was collected, filtered on 0.22 µm membrane (Millipore) and used immediately or stored at-20°C.

### Maintenance of the YL-1 (SCL-19) cell line

The YL-1 (SCL-19) cell line was cultured in 90% DMEM containing 10% fetal calf serum, 1 mM glutamine and 0.1 mM β-mercaptoethanol. This medium is called YL-1 (SCL-19) medium. Cells were incubated at 37°C in humidified air incubator gassed with 5% CO₂. Routine passage of the cells was performed once a week at subconfluence.

### Preparation of YL-1 (SCL-19) conditioned medium (YCM)

YL-1 (SCL-19) cells were seeded in T75 or T150 at 4.10⁴ cells/cm² in the YL-1 (SCL-19) medium. 24 hours later, the cells were irradiated with 1200 rad γ-irradiation and the medium was immediately replaced with KOSR medium. This treatment inhibits cell proliferation but does not affect the synthesis and secretion of factors important for hESC differentiation into endoderm. The next day, the medium was replaced again with fresh KOSR medium (40 ml for a T75). 48 hours later, the medium was harvested, filtered on a 0.22 µm membrane (Millipore) and used subsequently or stored at -20°C. At all times, cells were incubated at 37°C in humidified air incubator gassed with 5% CO₂.

### Maintenance and maturation of the primitive liver epithelial cell line

Cells were initially maintained in MCM medium in 6-well culture plates coated with collagen I at 8 µg/cm². For further maturation, cells were transferred to 6-well culture plates coated with collagen I at 8 µg/cm² in 90% DMEM containing 10% fetal calf serum, 1% non-essential amino-acids, 1 mM sodium pyruvate, 2 mM glutamine, 1% insulin/transferrin/selenium (Gibco) and 0.1 mM β-mercaptoethanol. This medium is called maturation medium. Cells were passaged once a week at subconfluence. Cells were incubated at 37°C in humidified air incubator gassed with 5% CO₂.

### Immunocytochemistry

Round glass slides were first treated with gelatin (1 mg/ml) for 30 minutes at room temperature then placed in the wells of a 12-well plate (Corning). Subconfluent or confluent cell cultures grown on those slides were fixed in 3% paraformaldehyde for 15 minutes at room temperature and then incubated in 50 mM ammonium chloride for 10 minutes. Cells were subsequently permeabilized in 0.2% Triton X-100 for 4 minutes. Fixed cells were then washed several times with PBS without calcium and magnesium and blocked in 0.5% BSA (bovine serum albumin) and PBS (BSA/PBS) for at least 5 minutes. Fixed cells were then incubated with primary antibodies used at the following dilutions in BSA/PBS for at least 1 hour at 4°C : antibody raised against albumin was used at 1:100 (ICN) and was directly coupled to FITC, AFP at 1:20 (R&D), Sox-17 at 1:20 (R&D), GATA-6 at 1:10 (R&D), α1-antitrypsin at 1:50 (Zymed), CK8 at 1:20 (Chemicon), CK18 at 1:20 (CBL), CK19 at 1:20 (Chemicon), c-met at 1:25 (R&D) and vimentin at 1:15 (Immunotech). Cells were washed three times with BSA/PBS and incubated with the following secondary antibodies coupled to various fluorochromes for 30 minutes at 4°C in the dark : antibodies coupled to FITC (fluorescein iso thio cyanate) were used at 1:50 (Becton Dickinson) and antibodies coupled to Alexa 546 were used at 1:800 (Molecular Probes). Cells were then washed three times with BSA/PBS then nuclei were stained with DAPI (4',6-Diamidino-2-phenylindole) at 125 ng/ml for 2 minutes then washed extensively with PBS. Stained cells were then mounted in Mowiol (Calbiochem). Images were taken at 20X and 40X using an epifluorescence microscope coupled to a CCD camera.

### Flow cytometry

A single-cell suspension was obtained after treating the cells for 5-10 minutes with a solution containing 0.2 mg/ml EDTA and 1 mg/ml BSA fraction V with PBS at 37°C. Cells were washed once with PBS containing 3% SR (FACS buffer). All the stainings were performed in FACS buffer. Primary antibodies were added to the cells at the following dilutions: CD49f was used at 1:10 (Chemicon), c-met at 1:25 (R&D), CD29 at 1:25 (Chemicon), CD54 at 1:25 (Chemicon), CD56 at 1:25 (Becton Dickinson), CD90 at 1:50 (Becton Dickinson) and SSEA-3 at 1:12 (DHSB) and incubated for 30 minutes on ice. Cells were then washed once with FACS buffer. Secondary antibodies coupled to specific fluorochromes were added to the cells at the following dilutions : antibodies coupled to FITC were used at 1:100 (Becton Dickinson) and antibodies coupled to PE were used at 1:100 (Becton Dickinson) and incubated for 20 minutes on ice. Cells were subsequently washed once with FACS buffer and resuspended in FACS buffer containing propidium iodide at 1.5 µg/ml. Events were monitored on a LSR flow cytometer (Becton Dickinson) and data were analyzed using CellQuest software (Becton Dickinson).

### Dosage of secreted proteins

For albumin dosage, the proteins in the sample form immune complexes with specific antibodies. These complexes scatter a beam of light passed through the sample. The intensity of the scattered light is proportional to the concentration of the relevant protein of the sample. The result is evaluated by comparison with a standard of known concentration (N antiserum to human albumin, prealbumin and retinol-binding protein, No. OSAL, Dade Behring Inc, Germany

For alpha-fetoprotein dosage, the proteins in the sample are detected using AFP coated with magnetic microparticles. The conjugate is then detected by chemoluminescence (Architect System, Ref 6C01, Abbott, Germany)

### Examples

A cell culture system to derive a heterogenous population of endodermal cells from human embryonic stem cells is described. The differentiation process involved is direct, which means that it is obtained in a non-random fashion without the use of embryoid bodies. The obtained differentiated cell population can be defined by a particular morphology and the expression of specific markers detailed below. The obtained differentiated cell population can be further driven to commit, under specific cell culture conditions, into a more homogenous primitive liver epithelial stem cell type (progenitor cell type).

It has been discovered that when pluripotent human embryonic stem cells are cultured in the presence of the conditioned medium of the YL-1 (SCL-19) cell line, the morphology and spatial organization of the cells studied by inverted microscopy begins to change starting from day 4, when compared to the same cell type under self-renewing conditions. This typical and recognizable differentiation process with the formation of typical crests and cavities becomes much more pronounced with time (between day 7 and day 12).

Ultrastructure of the differentiating colonies analyzed by electron microscopy revealed the presence of a homogenous population of cells in terms of morphology with characteristics of immature cells of the hepatocyte lineage : glycogen granules, high nucleo-cytoplasmic ratio, the ability of cells to form tight intercellular junctions resulting in the creation of bile canaliculi-like structures.

Phenotypic analysis of the differentiated cells by immunocytochemistry showed that they express two endoderm-specific nuclear transcription factors, namely Sox-17 and GATA-6 as well as α-foetoprotein (AFP) and albumin, two intracellular and secreted proteins whose expression is correlated with a liver cell phenotype.

Furthermore, flow cytometry studies confirmed that under the above-mentioned cell culture conditions, hESCs undergo a the process of differentiation because the SSEA-3 marker (a typical cell surface marker known to be expressed only by pluripotent self-renewing hESCs) is down-regulated.

Moreover, flow cytometry studies demonstrated that within the differentiating colonies a subset of cells express one or more of the following markers : CD49f, CD29, CD56, and CD54. These markers are reminiscent of a primitive liver epithelial stem cell phenotype like the one described for hepatoblasts or for fetal liver bipotential progenitors.

The described system therefore results in the production of a heterogenous population of cells in terms of phenotype that contains a subset of cells with phenotypic characteristics of primitive liver epithelial stem cells.

However, other primitive endodermal stem cell types may be present within the described heterogeneous population for example primitive pancreatic stem cells, primitive gut stem cells, primitive thymic stem cells, primitive lung stem cells etc.

It is also possible to derive from the above-mentioned heterogenous population a more homogenous population of primitive epithelial liver stem cells displaying many characteristics of human hepatocyte progenitor cell lines.

The described system therefore allows the obtaining of a more homogeneous cell population which represents an almost unlimited supply of cells with a primitive liver epithelial stem cell phenotype.

It is also possible to obtain more mature cells derived from the primitive liver epithelial cell line when cultured in a medium containing fetal calf serum. The cells lose the expression of AFP but retain the expression of albumin, a feature of a more mature hepatic cell type.

### Example 1 : Direct differentiation of human embryonic stem cells using the YL-1 (SCL-19) conditioned medium

Mitomycin-treated mouse embryonic fibroblasts were seeded at 4.10⁴ cells/cm² on top of gelatinized round glass slides that have been placed on the surfaces of a 12-well support plate in KOSR medium. At what we consider as day 0 of differentiation, hESC colonies grown in 6-well culture plates in 3 ml of KOSR medium were scraped with a 10 µl pipette tip and the resulting hESC clusters (contained in 40-50 µl depending on hESC colonies density) were then placed on top of the MEFs in the presence of YL-1 (SCL-19) conditioned medium. The next day, considered as day 1 of differentiation, the culture medium was replaced with fresh YL-1 (SCL-19) conditioned medium. The medium was then replaced every 2 days.

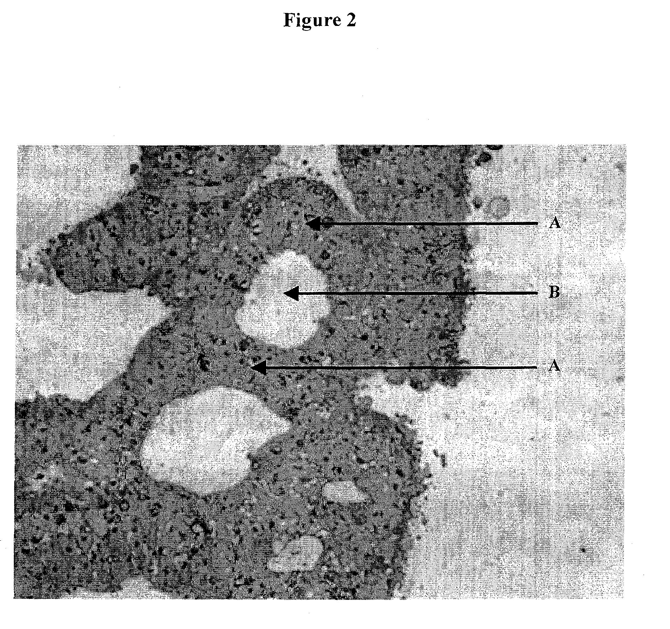

Starting from day 4, hESC colonies grown in the YL-1 (SCL-19) conditioned medium started to change morphologically when compared to colonies grown in KOSR medium (see Figure 1 ; see in particular picture 1G showing a hESC colony grown in the YL-1 (SCL-19) conditioned medium at day 7 vs. picture 1H showing a hESC colony grown in KOSR medium at day 7) and displayed a three dimensional arrangement consisting in the formation of typical dense cell clusters or crests distributed around cavities. The differentiation process became much more pronounced with time (between day 7 and day 12 up to day 20). On semi-thin sections, typical images were seen where cells composing the crests were distributed around cavities (Figure 2).

Seven days after plating, typical colonies containing crests and cavitie growing on glass slides were fixed and prepared for electron microscopy studies.

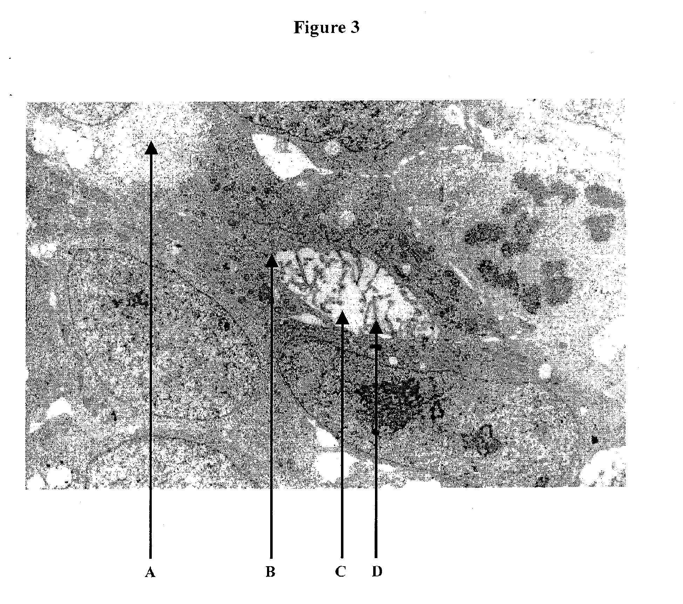

At day 7, the cells composing the crests were homogenous in terms of morphology and shared many characteristics with immature epithelial liver cells (Figure 3). The cells exhibited a large nucleo-cytoplasmic ratio and contained an immature nucleus without heterochromatin. The cells were often elongated or rounded and were arranged in monolayers and organized as cords. Numerous bile canaliculi-like structures exhibiting long microvilli were observed. These canaliculi were formed in general by two cells and sealed by typical intercellular tight junctions and desmosomes. Numerous junctional complexes were visible (tight and adherent junctions as well as desmosomes) which are typical of polarized cells. Typical organelles reminiscent of liver cells were observed like well developed Golgi apparatus with numerous dictyosomes, mitochondria and smooth endoplasmic reticulum with some rough endoplasmic reticulum. Most of the ribosomes were free and organized into polysomes suggesting that the cells do not have a major secretory activity but can store many intracellular proteins. The cells contained many typical fibrils suggesting the presence of cytokeratins. Lipid droplets were observed in the cytoplasm of many cells. Finally, many glycogen rosettes could be seen in the cytoplasm, a typical feature of hepatocytes.

### Example 2 : Markers expressed by differentiating hESC colonies

Differentiating cells derived from hESCs were fixed for immunohistochemistry using antibodies against various endoderm specific markers. Results are shown in Table 1. All the cells expressed AFP and albumin which is reminiscent of an endoderm origin. However no more than 50 % of the cells expressed antibody-detectable levels of either GATA-6 or SOX-17 suggesting the presence of more than one cell type in the differentiating colonies. The expression of these two transcription factors in conjunction with AFP and albumin strongly suggest that more than 50% of the cells are of definitive endodermal origin.

**Table 1: Immunocytochemistry of differentiating colonies**

| Specificity of primary antibodies | hESCs cultured for 7 days |
|---|---|
| (none) | 0% positive |
| non-specific IgG | 0% positive |
| AFP | 100% positive |
| Albumin | 100% positive |
| GATA-6 | 50% positive |
| SOX-17 | 50% positive |

### Example 3: Markers expressed by differentiating human cells

Differentiating cells derived from hESCs were harvested at day 3, 7 and 11 of differentiation for flow cytometry analysis of the following cell surface markers known to be associated with an epithelial liver stem cell or hepatoblast phenotype : CD49f (b1 integrin), CD29 (a6 integrin), CD54 (1-CAM) and CD56 (N-CAM). Results are shown in Figure 4.1 to 4.4. The numbers are expressed as a mean of four independent experiments.

### Example 4 : Dosage of secreted plasma proteins by differentiating cells

The conditioned medium of differentiating hESC colonies was harvested every 2 days and used for biochemistry analysis of secreted AFP. Results are shown in Figure 5 (alpha-fetoprotein secretion vs. time).

### Example 5 : Derivation of a primitive liver epithelial cell line

The differentiation process was induced as previously described. Colonies showing a typical morphology at day 13 of differentiation were harvested and pooled. Colonies were treated with DispaseII 10 mg/ml for 5mn at 37°C to obtain a single-cell suspension, washed once with PBS then seeded in a 6-well plate coated with collagen I at 8 µg/cm2 and containing MCM medium. After 24 hours, some cells spread then began to proliferate. The cells had a fibroblastoid-like morphology with bright refractile borders and had a tendency to scatter within a few days. After a week of culture, growing cells had formed epithelial clusters of cells with a polygonal shape morphology and could be serially passaged using Dispase II and the same culture conditions. See figure 6 for a reference. At confluence, the cells had an epithelioid morphology and binucleated cells were often seen (picture 6G).

At several passages, cells were harvested and analyzed by immunohistochemistry for enoderm and immature and mature liver cell specific markers. Mouse embryonic fibroblasts (MEFs) were used as a negative control and the human hepatoma cell line HepG2 as a positive control for immunofluorescence staining. Results are shown in Table 2.

**Table 2: Immunohistochemistry of the primitive cell line**

| | passage 1 | passage 4 | passage 6 | HepG2 | MEFs |
|---|---|---|---|---|---|
| AFP FITC | + | + | ++ | +++ | - |
| Alb FITC | + | + | ++ | +++ | +/- |
| CK8 FITC | ND | ND | + | ++ | +/- |
| CK 18 FITC | + | ND | ++ | +++ | +/- |
| CK19 546 | ND | ND | +/- | - | - |
| α-AAT 546 | ND | ND | + | +++ | +/- |
| GATA-6 FITC | - | +/- (cytosol) | ND | ND | ND |
| Sox-17 546 | +++ (cytosol) | ND | ND | ND | ND |
| Vimentin FITC | +++ (60%) | +/- | ND | ND | ND |

| | | | | | |
|---|---|---|---|---|---|
| -: no fluorescence, +/-: background fluorescence, +: low fluorescence , ++: high fluorescence, +++: very high fluorescence, ND: not determined. | | | | | |

Vimentin is a mesodermal marker that has been described to be expressed by human fetal liver ductal plate and biliary epithelial cells and porcine liver epithelial cell lines (Haruna et al, 1996, Hepatology, 23, 3, 476-481 ; Kano et al, 2000, Am. J. Pathol., 156, 6, 2033-2043).

In conclusion, the derived cell line has an epitheloid morphology and express many markers reminiscent of primitive liver cells.

### Example 6 : Other markers expressed by the primitive liver epithelial cell line

The primitive liver cell line was studied by flow cytometry using antibodies associated with a liver stem cell or hepatoblast phenotype as well as markers specific of a more differentiated liver phenotype. Results are shown in Table 3.

**Table 3: Phenotype of the primitive liver cell line**

| Specificity of primary antibody | Passage 1 |
|---|---|
| (none) | 0% |
| non-specific IgG | 0% |
| c-met | 20% |
| CD49f | 8% |
| CD29 | 16% |
| CD 54 (1-CAM) | 7% |
| CD 56 (N-CAM) | 17% |
| CD90 (Thy-1) | 14% |

### Example 7 : Further maturation of the primitive liver epithelial cell lines

When transferred to maturation medium, the primitive liver epithelial cells adopted a polygonal shape and formed rosette-like clusters in particular when reaching confluence.

Figure 7 shows the typical morphology of the matured primitive liver epithelial cell line.

## Claims

1. The use of the YL-1 (SCL-19) cell line, deposited at CNCM under No. 1-3348 on December 23, 2004, for deriving human primitive endodermal stem cells and / or human primitive epithelial liver cells and / or human matured epithelial liver cells in vitro from human pluripotent stem cells, in particular from human embryonic stem cells.

2. A population of human primitive endodermal stem cells, containing cells expressing albumin, alpha-fetoprotein, and at least one of GATA-6 and SOX-17.

3. The population of human primitive endodermal stem cells of claim 2, wherein the SSEA-3 marker is down-regulated relative to undifferentiated pluripotent human embryonic stem cells.

4. The population of human primitive endodermal stem cells of claim 2 or 3, wherein at least 90 % of the cells of said population express albumin and at least 90 % of the cells of said population express alpha-fetoprotein and at least about 40 % of the cells of said population express GATA-6 and at least about 40 % of the cells of said population express SOX-17.

5. The population of human primitive endodermal stem cells of one of claims 2 to 4, comprising cells which express one or more of the following markers : CD29, CD49f, CD54 and CD56.

6. The population of human primitive endodermal stem cells of claim 5, comprising cells which express CD29, CD49f and CD56.

7. The population of human primitive endodermal stem cells of one of claims 5 or 6, further **characterized in that** :
more than about 50 %, in particular from about 70 % to about 80 %, in particular about 75 % of the cells of said population express CD29 and / or
more than about 60 %, in particular from about 70 % to about 85 %, in particular about 75 % of the cells of said population express CD49f and / or
more than about 1 %, in particular from about 1 % to about 3 %, in particular about 2 % of the cells of said population express CD54 and /or
more than about 15 %, in particular from about 40 % to about 60 %, in particular about 50 % of the cells of said population express CD56.

8. A population of human primitive epithelial liver stem cells, which is substantially homogenous and wherein the cells of said population have the property of being able to multiply without any substantial change of phenotype for at least 20 passages and at least part of those cells express alpha-fetoprotein, albumin, CK 18, CD56 and c-met.

9. The population of human primitive epithelial liver stem cells of claim 8, further **characterized in that** one or more of the following markers are also expressed : CK8, alpha-AAT, vimentin, SOX-17, CD49f, CD29, CD54 and CD90.

10. A human primitive endodermal stem cell, **characterized in that** it expresses alpha-fetoprotein, albumin, SOX-17 and GATA-6.

11. The human primitive endodermal stem cell of claim 10, further **characterized in that** it expresses one or more of the following markers : CD29, CD49f, CD54 and CD56.

12. The human primitive endodermal stem cell of claim 10, further **characterized in that** it expresses CD29, CD49f, CD45 and CD56.

13. A human primitive epithelial liver stem cell, **characterized in that** it expresses alpha-fetoprotein, albumin, CK18, CD54 and c-met.

14. A process for preparing primitive endodermal stem cells in vitro, comprising :
― the incubation of a culture of human embryonic stem cells in a YL-1 (SCL-19) conditioned medium derived from a culture of YL-1 (SCL-19) cells from the YL-1 (SCL-19) cell line deposited at CNCM under No. I-3348 on December 23, 2004, for an amount of time appropriate for the differentiation of said human embryonic stem cells into primitive endodermal stem cells, and
― the recovery of said primitive endodermal stem cells from said YL-1 (SCL-19) conditioned medium.

15. The process of claim 14, wherein said YL-1 (SCL-19) conditioned medium is obtained by :
- irradiating a culture of YL-1 (SCL-19) cells from the YL-1 (SCL-19) cell line deposited at CNCM under No. I-3348 on December 23, 2004,
- further culturing the YL-1 (SCL-19) cells in a primary YL-1 (SCL-19) culture medium to obtain a YL-1 (SCL-19) conditioned medium, and
- recovering said YL-1 (SCL-19) conditioned medium.

16. The process of claim 14 or 15, wherein said amount of time appropriate for the differentiation of said human embryonic stem cells into primitive endodermal stem cells is at least 4 days, in particular 4 to 20 days.

17. The process of one of claims 14 to 16, comprising :
- the culture of human embryonic stem cells in KOSR medium,
- the incubation of a sample of said human embryonic stem cells on a culture of mitomycin-treated mouse embryonic fibroblasts in the YL-1 (SCL-19) conditioned medium, said YL-1 (SCL-19) conditioned medium being obtained by
• seeding YL-1 (SCL-19) cells from the YL-1 (SCL-19) cell line deposited at CNCM under No. 1-3348 on December 23, 2004 in a primary YL-1 (SCL-19) culture medium such as the one comprising 90 % Dulbecco's modified Eagle's medium, 10 % fetal calf serum, 1 mM glutamine and 0.1 mM betamercaptoethanol,
• irradiating by gamma irradiation said YL-1 (SCL-19) cells,
• replacing said YL-1 (SCL-19) culture medium by KOSR medium,
• filtering said KOSR medium after about 2 days after said seeding,
said incubation lasting for an amount of time appropriate for the differentiation of said human embryonic stem cells into primitive endodermal stem cells, said amount of time being at least 4 days, in particular 4 to 20 days, and
- the recovery of said primitive endodermal stem cells from said YL-1 (SCL-19) conditioned medium.

18. A YL-1 (SCL-19) conditioned medium such as obtained by :
- irradiating a culture of YL-1 (SCL-19) cells from the YL-1 (SCL-19) cell line deposited at CNCM under No. 1-3348 on December 23, 2004,
- further culturing the YL-1 (SCL-19) cells in a primary YL-1 (SCL-19) culture medium to obtain a YL-1 (SCL-19) conditioned medium, and
- recovering said YL-1 (SCL-19) conditioned medium.

19. A population of primitive endodermal stem cells such as prepared in vitro according to the process of one of claims 14 to 17.

20. A process for preparing primitive epithelial liver stem cells in vitro, comprising
- the culture of primitive endodermal stem cells prepared according to the process of one of claims 14 to 17 for a sufficient amount of time in the presence of MCM medium so that said primitive endodermal stem cells differentiate into primitive epithelial liver stem cells, and
- the recovery of said primitive epithelial liver stem cells from said MCM medium.

21. The process of claim 20, wherein said MCM medium is obtained through a process comprising culturing fibroblasts in a culture medium and then recovering said culture medium.

22. The process of claim 20 or 21, wherein said sufficient amount of time is approximately one week.

23. The process of one of claims 20 to 22, comprising :
- the preparation of primitive endodermal stem cells according to the process of one of claims 14 to 17,
- the treatment of said primitive endodermal stem cells with dissociating means, the seeding of said cells on culture plates coated with collagen in MCM medium,
- the culture of said primitive endodermal stem cells for about one week, so that said primitive endodermal stem cells differentiate into primitive epithelial liver stem cells,
- the optional passaging of said primitive epithelial liver stem cells once or several times using a detaching means such as DispaseII and said MCM medium, and
- the recovery of said primitive epithelial liver stem cells from said MCM medium.

24. A population of primitive epithelial liver stem cells such as prepared according to the process of one of claims 20 to 23.

25. A process for preparing matured epithelial liver cells in vitro, comprising :
― the culture of primitive epithelial liver stem cells prepared according to the process of one of claims 20 to 23 in a maturation medium so that said primitive epithelial liver stem cells differentiate into matured epithelial liver cells, and
― the recovery of said matured epithelial liver cells from said maturation medium.

26. The process of claim 25, comprising :
- the preparation of primitive epithelial liver stem cells according to the process of one of claims 20 to 23,
- the culture of said primitive epithelial liver stem cells in a maturation medium comprising 90 % Dulbecco's modified Eagle's medium, 10 % fetal calf serum, 1 % non-essential amino-acids, 1 mM sodium pyruvate, 2 mM glutamine, 1% insulin / transferrin / selenium, so that said primitive epithelial liver stem cells differentiate into matured epithelial liver cells, and
- the recovery of said matured epithelial liver cells from said maturation medium.

27. A population of matured epithelial liver cells such as prepared according to the process of claim 25 or 26.

28. The use of a population of cells, said population of cells being the population of primitive endodermal stem cells of any one of claims 2 to 7 and 19 or the population of primitive epithelial liver stem cells of any one of claims 8, 9 and 24 or the population of matured liver stem cells of claim 27, or of the cells of one of claims 10 to 13, for carrying out toxicological tests comprising :
- submitting said population of cells or said cells to a substance having a possible toxic effect on said population of cells or said cells, and
- assessing the toxicity of said substance on said population or said cells.

29. The use of a population of cells, said population of cells being the population of primitive endodermal stem cells of any one of claims 2 to 7 and 19 or the population of primitive epithelial liver stem cells of any one of claims 8, 9 and 24 or the population of matured liver stem cells of claim 27, or of the cells of one of claims 10 to 13, for carrying out drug screening comprising :
- submitting said population of cells or said cells to a substance having a possible therapeutic or prophylactic effect on humans or animals,
- either before or after said submitting to said substance, submitting said population of cells or said cells to a treatment for placing said population or said cells in a substantially pathological state, and
- assessing the possible therapeutic or prophylactic effect of said substance on said population of cells or said cells placed in a substantially pathological state.

30. A pharmaceutical composition comprising as active substance a population of cells, said population of cells being the population of primitive endodermal stem cells of any one of claims 2 to 7 and 19 or the population of primitive epithelial liver stem cells of any one of claims 8, 9 and 24 or the population of matured liver stem cells of claim 27, or the cells of one of claims 10 to 13, in association with a pharmaceutically acceptable carrier.

31. The use of a population of cells, said population of cells being the population of primitive endodermal stem cells of any one of claims 2 to 7 and 19 or the population of primitive epithelial liver stem cells of any one of claims 8, 9 and 24 or the population of matured liver stem cells of claim 27, or of the cells of one of claims 10 to 13, for preparing a medicament for the treatment of cancers or of genetic pathologies or of hepatic disorders, in particular for the treatment of inherited hepatic disorders such as Wilson's disease, alpha-1 antitrypsin deficiency, erythropoietic protoporphyria, Niemann-Pick disease or type I tyrosinemia or of acquired hepatic disorders such as end-stage cirrhosis and liver-based congenital metabolic diseases.
